# EUROPEAN PATENT SPECIFICATION

(11) **EP 0 293 488 B1**
(45) Date of publication and mention of the grant of the patent: **28.07.1993**
(21) Application number: 87907824.4
(22) Date of filing: 27.11.1987
(51) Int. Cl.: A01G 1/00, C12N 5/00

(54) **METHOD OF MULTIPLYING TUBERS**
VERAHREN ZUR VERMEHRUNG VON KNOLLEN
PROCEDE DE MULTIPLICATION DE TUBERCULES

(30) Priority: 02.12.1986 JP 287213/86
(43) Date of publication of application: 07.12.1988
(73) Proprietor: KYOWA HAKKO KOGYO CO., LTD., Chiyoda-ku, Tokyo-to (JP)
(72) Inventor: TAKAYAMA, Shinsaku, Ibaraki 305 (JP); AKITA, Motomu, Ibaraki 305 (JP)
(74) Representative: Lambert, Hugh Richmond
(86) International application number: PCT/JP87/00923
(87) International publication number: WO 88/04136

(56) References cited:
- EP-A- 0 098 234
- JP-A-55 118 319
- JP-B- 5 543 723
- US-A- 4 115 950
- "Potato physiology" 1985, Academic Press Inc, New York Ch.15 :P.Wang & C:Hu Potato tissue culture and its application in agriculture pp 503-577.
- American Potato Journal vol. 60, 1983, pages 27 - 33; G.Wattimena et al: "Comparative fieldperformance of potatoes from microculture"
- American Potato Journal vol. 59, 1982, && C.Hu: "In vitro mass tuberization and virus-free seed potato production in Taiwan"
- Annals of Botany vol. 53, 1984, &&bN.J.Stacey: "Factors affecting the formation of in vitro tubers of potato (Solanum tuberosum L.)"
- American Potato Journal, Vol. 55, p. 691-701, 1978
- Soshiki baiyo, Vol. 11, No. 9, p. 391-395, p. 372-376, 1985 (Japan)

## Description

This invention relates to a method for the mass propagation of tuber-forming plants selected from the group consisting of potato, taro, konjak, jack-in-the-pulpit, Scopolia japonica and Chinese yam, and especially potatoes.

A variety of tissue culture methods have been proposed for the propagation of potatoes.

It is known, for example, to obtain potato tubers by culturing potato tissue to form a potato plant, removing roots and leaves from the resulting plant and subjecting the stem to liquid culture to produce the tuber: CIP Circular, vol.13, No. 4, December, pages 1 to 5 (1985); and Plant Cell, Tissue and Organ Culture, 7 : 3 to 10 (1986). In this method, the culture is performed in a flask in a thinly spread layer of culture medium so that the tubers are formed in the aerial phase. The number of tubers produced is however, not large.

It is also known to propagate potato tubers in a culture medium with repeated division of the tuber: American Potato Journal, 55: 691 - 7-1 (1978). However, even in the most efficient method, only 140 plants were obtained in 16 weeks.

Small-sized potato tubers weighing 0.1 to 0.7g have also been obtained by the method disclosed in: Soshiki Baiyo (Tissue Culture). 11 (9), 391 - 395, 1985.

The culture was performed in a small-sized container using a single medium and took approximately 100 days to produce the tubers and is inherently unsuitable for operation on a larger scale.

Taro, Chinese yam and jack-in-the-pulpit have been propagated by plant tissue culture techniques: Yasai Shikenjo Hokoku A (Report A of the Vegetable Laboratories), 9 : 1 - 46 (1981), Soshiki Baiyo (Tissue Culture), 11 : 372 - 376 (1985), but with low efficiency.

The need still exists, therefore, for an efficient tissue culture method for the propagation of potato, taro, konjak, jack-in-the-pulpit, Scopolia japonica and Chinese yam tuberous root tissues, in large quantities.

According to the present invention, there is provided an improved method for the mass propagation of tuber-forming plants selected from the group consisting of potato, taro, konjak, jack-in-the-pulpit, Scopolia japonica and Chinese yam by tissue culture, which comprises:
A) aseptically culturing in a culture medium and to an initial plant size not exceeding 10 cms plant tissue from the selected species, such tissue containing at least one growth point;
B) aseptically subdividing the cultured plant material obtained in step A) and transferring the subdivided material to a fresh liquid culture medium containing a suitable carbon source, at a concentration of 60 g/L or less;
C) continuing the culture of the subdivided plant material under an illuminance of 200 to 10,000 lux in the fresh culture medium to obtain a cultured plant material having an average plant size in the range 15 to 30 cms;
D) in the event that 90% of the cultured plant material obtained in step C) is not submerged in the culture medium, adding fresh culture liquid until 90% of the cultured plant material is so submerged;
E) increasing the concentration of the carbon source in the liquid culture medium to 60 to 150 g/L and continuing the culture, but under an illuminance of 200 lux or less;
F) progressively reducing the volume of the culture liquid throughout step E) until from 50 to 98% of the cultured plant material is above the level of the culture medium, thereby to establish a propagated plant material comprising the tubers, corms, rhizomes or other tuberous root tissue of the plant in question and located substantially in the air phase above the culture liquid;
G) recovering from the plant culture at the end of step F) the said tubers, corms, rhizomes or other tuberous root tissue.

Hereinafter the invention will be described largely with respect to potatoes, although it is to be understood that the techniques, conditions and materials described apply also to the culture of the other plants named, viz: taro, konjak, jack-in-the-pulpit, Scopolia japonica and Chinese yam.

Referring to the mass propagation of potatoes by the method of the present invention, an initial culture is first established by aseptically culturing tissue from a potato plant, e.g. stem, leaf, root, or tuber tissue which contains a growing point, to an initial plant size not exceeding 10 cms. The plant material is then subdivided and transplanted into fresh liquid culture medium, preferably at from 5 to 50 pieces/l, for continued growth under the conditions to be described in more detail hereinafter.

For each of the culture steps, any suitable culture medium, natural or synthetic, can be used as long as it contains the necessary sources of carbon and nitrogen sources and inorganic materials, etc.

Suitable carbon sources, include sucrose, maltose, fructose, glucose, molasses, etc.

Nitrogen sources include potassium nitrate, sodium nitrate, ammonium nitrate, calcium nitrate, ammonium sulfate, amino acids (glycine, glutamic acid, lysine, aspartic acid, etc.), yeast extract, meat extract, peptone, coconut milk, etc.

Inorganic materials present in the culture media may include any of the following: potassium chloride, calcium chloride, manganese chloride, nickel chloride, cobalt chloride, aluminum chloride, iron chloride, magnesium sulfate, sodium sulfate, nickel sulfate, iron sulfate, manganese sulfate, zinc sulfate, copper sulfate, monopotassium phosphate, potassium iodide, boric acid, sodium molybdenate, etc.

If necessary, and indeed usually, the media will also contain other growth factors, including, for example one or more of cytokinin, benzyl adenine (hereinafter referred to as BA), N-(2-chloro-4-pyridine)-N-phenylurea (hereinafter referred to as 4PU), kinetin, chlorocholine hydrochloride, abscisic acid, vitamin B₁, inositol, pyridoxine hydrochloride, nicotinic acid, thiamine hydrochloride and biotin, etc.

Typically suitable culture medium are, for example, Murashige-Skoog medium, Erickson medium, White medium, Linsmayer-Skoog medium etc.

The preferred carbon sources are glucose and sucrose, which in step B are used at a first concentration of 60 g/L or less, whilst in step E the further culture is carried out under an illuminance of 200 lux or less.

After initial cultivation and subdivision cultivation is continued in fresh culture medium in which the concentration of the carbon source is 60 g/l or less, preferably 25 to 35 g/l and at a medium depth of 12 cms or less, preferably 5 to 10 cms, a temperature of 10 to 35°C, an illuminance of 200 to 10,000 lux, a pH of 4 to 8, and an aeration rate of 0.01 to 0.5 vvm, preferably 0.05 to 0.2 vvm, until the average length of the cultured plant material is 15 to 30 cms. In general, this takes 20 to 60 days.

At this stage, culture conditions are changed in order to induce the growth of tubers.

Initially, and as may or may not be necessary, fresh medium is added to the culture until at least 90% of the cultured plant material is below the surface. The concentration of the carbon source, which is preferably sucrose, is then increased to 60 to 150 g/l, preferably 80 to 100 g/l. Cultivation then continues at a temperature of 10 to 35°C, an illuminance of 200 lux or less, preferably in the dark and a pH of 4 to 8 and with progressive removal of culture liquid until 50 to 98% of the cultured plant material is above the culture medium. In general, this takes from 20 to 90 days.

The decrease in the volume of the liquid medium with time, may be achieved in various ways. For example, the rate of evaporation of the culture liquid may be increased by aeration of the culture. Alternatively, the liquid medium may simply be decanted, or a combination of both may be used.

Where the volume of the liquid medium is to be decreased by evaporation, the aeration rate may be increased to a value in the range 0.8 to 2.2 vvm, which is higher than that used in Step (A). Where liquid medium is directly decanted, this is preferably done in a series from 3 to 10 steps, starting when the formation of potato tubers is observed around the interface between the medium and the aerial phase. At this stage from 10 to 20%, for example, of the medium may be discharged.

At the end of the culture potato tubers are obtained, many of which have a weight of 1 g or more.

In the case of the other species: taro, konjak, jack-in-the-pulpit, Scopolia japonica and Chinese yam, culture is performed in exactly the same way as described above for potato cultures.

The method of the invention is illustrated by the following examples.

### Example 1

Potato corms were planted into 10 ml of a sterilised (at 121°C for 20 minutes) Murashige-Skoog medium having the composition given in Table 1, in a test tube having a diameter of 25 mm and a length of 125 mm, and cultivated at 25°C under continuous lighting conditions (2500 lux) for about a month by which time 20 potato plants were obtained grown to a size of about 10 cms. The plants were then divided at every joint and transferred into a 16l jar fermenter containing 5l of liquid medium to a depth of 9 cms. The liquid medium has the composition shown in Table 1 but without the agar. Cultivation was performed at 25°C under continuous lighting (4000 lux) for three weeks with continuous aeration at the rate of 0.1vvm. By the end of three weeks the plants had attained an average length of 20 cms. The culture medium was then totally replaced by 10L of fresh liquid medium of the same composition shown in Table 1 but without the agar and with a sucrose concentration of 9% (w/v).

The depth of the medium in the fermenter was now 18 cms. Cultivation continued at 25°C at an aeration rate of about 0.1 vvm, but in total darkness. After five days tuber formation was noted, mainly around the interface between the medium and the aerial phase. 2l of the culture medium were then decanted to lower the liquid surface and the cultivation continued. Four further 2l quantities of medium were discharged at 5 day intervals thus progressively lowering the culture level.

At the end of 45 days continuous culture the remaining amount of culture liquid was 0.8l at a depth of 1 cm. At this stage about 95% of the cultured plant material was exposed to the aerial phase but not blighted. About 370 tubers in total were collected from the aerial phase and from the interface of the medium with the aerial phase. Of these, 80 weighed 1 g or more.

**Table 1**

| Murashige-Skoog Medium | |
|---|---|
| Ammonium nitrate | 1650 mg |
| Potassium nitrate | 1900 mg |
| Calcium chloride dihydrate | 440 mg |
| Magnesium sulfate heptahydrate | 370 mg |
| Potassium dihydrogenphosphate | 170 mg |
| Na₂.EDTA dihydrate | 37.3 mg |
| Ferric sulfate heptahydrate | 27.8 mg |
| Boric acid | 6.2 mg |
| Manganese sulfate tetrahydrate | 22.3 mg |
| Zinc sulfate heptahydrate | 8.6 mg |
| Potassium iodide | 0.83 mg |
| Sodium molybdenate dihydrate | 0.25 mg |
| Cuprous sulfate | 0.025 mg |
| Cobalt chloride | 0.025 mg |
| Vitamin B₁ | 0.40 mg |
| Inositol | 100 mg |
| Pyridoxine hydrochloride | 0.50 mg |
| Nicotinic acid | 0.50 mg |
| Glycine | 2.00 mg |
| Sucrose | 30.0 g |
| Agar | 8.0 g |
| Deionised water | to 1 litre |
| 0.1N NaOH | to pH 6.2 |

### Example 2

Potato corms were planted into 10 ml of a Murashige-Skoog medium having the composition given in Table 1, in a test tube having a diameter of 25 mm and a length of 125mm, and cultivated at 25°C under continuous lighting conditions (2500 lux) until the plant material had grown to about 10 cms. The plant material was then divided at every joint to obtain 100 pieces which were then transferred into a 8l-jar fermenter containing 2l of fresh liquid medium having the composition shown in Table 1 but without the agar, and to a depth of 8 cms. Cultivation was performed at 25°C under continuous lighting conditions (4000 lux) for four weeks with continuous aeration at the rate of 0.1 vvm. At the end of four weeks the plants had grown to an average length of 25 cms. The culture medium was then completely replaced by 6l of fresh liquid medium of the composition shown in Table 1 but without the agar and with an increased sucrose concentration of 9% (w/v); depth of medium: 23 cms. Cultivation was continued in the dark at 25°C for 4 weeks at an aeration rate of 0.8 vvm. At the end of that time approximately 2l of culture medium remained to a depth of 8 cms with approximately 70% of the cultured plant material exposed in the aerial phase. About 390 potato tubers were obtained, total weight above 300g, Of those 110 tubers weighted 1 g or more.

In a separate experiment cultivation was performed in a similar manner except that the aeration rate was reduced to 0.1 vvm. The amount of the medium remaining at the end of the culture was approximately 5.2l to a depth of 19 cms. 250 potato tubers were obtained, total weight about 210g. Of those, 80 tubers weighed 1 g or more.

### Example 3

Tissue slices containing growth points were obtained from the corm buds of taro (species: Ishikawa Wase) by dissection under a stereoscopic microscope under aseptic conditions. The collected tissue slices were then placed into 10 ml of a Murashige-Skoog medium having the composition given in Table 1 in a test tube having a diameter of 25 mm and a length of 125 mm. Those slices were cultivated at 25°C under continuous lighting conditions (2500 lux) for about 3 months. The obtained plants were then transferred into 300 ml-flask containing 100 ml of fresh liquid medium of the composition given in Table 1 but without the agar. Rotary spinner culture (180 r.p.m.) was performed at 25°C under continuous lighting conditions (2500 lux). After two months of continuous culture, 250 plants were obtained. These were aseptically removed from the flask, divided into 10 groups of 25 plants each of which groups was transplanted into a 300 ml-flask containing 100 ml of fresh liquid medium having the same composition as described above. Rotary spinning culture (180 r.p.m.) was carried out on each flask at 25°C under continuous lighting conditions (2500 lux) for one month to propagate the plants. At the end of one month, the propagated plants in each flask were again divided into 10 groups, and each group again transferred to a new flask containing fresh culture medium, and cultured for a further month under the same culture conditions, the whole operation being repeated 20 times. During this repetitive cycle the number of plants in each flask gradually increased to a final average density of 700 plants per flask. The contents of 3 flasks were then transplanted to a 8l-jar fermenter containing 6l of fresh liquid medium to a depth of about 23 cms, that liquid medium having essentially the same composition as before but with the sucrose concentration increased to 9% (w/v). Cultivation was continued for a further month at 25°C at an aeration rate of 0.1 vvm under conditions of continuous lighting (2500 lux) until the plants achieved an average length of 23 cms. The aeration rate was then increased to 2 vvm and the culture continued for a further month. During this period the liquid medium gradually evaporated so that the propagated plants were progressively exposed into the aerial phase above the culture medium. At the end of one month 1.2l of liquid culture medium remained to a depth of 5 cms. At this stage 80% of the culture plant material was in the aerial phase, but not blighted. At this stage the plants were taken out of the fermentation vessel and inspected. The basal parts of almost all plants had thickened and formed corms, with approximately 2900 corms from each fermenter. The total weight of the corms produced was 0.8 kg, with an average corm weight of 0.3g. 370 corms were obtained weighing in excess of 1 g. In contrast, the final stages of the culture were repeated on a separate batch of propagated plants but without changing the rate of aeration which remained at 0.1 vvm. At the end of two months 4.7l of culture medium remained to a depth of 18 cms. In this case only 430 taro corms were obtained per fermenter, total corm weight: 130 g., average weight of each corm: 0.3 g. Only 65 corms were obtained weighing 1 g or more.

### Example 4

Konjak corms were wrapped in water-absorbing paper impregnated with 500 ppm of BA and further wrapped in a vinyl sheet to prevent moisture loss by evaporation. After standing at 25°C for 7 days each corm developed about 100 axillary buds to a size of 3 to 10 mm. From these buds the growth points were aseptically dissected under a stereoscopic microscope and asceptically collected. The dissected growth points were then transplanted into 10 ml of a Murashige-Skoog medium having the composition given in Table 1 but supplemented with 4 PU in a concentration of 1 mg/l, and contained in a test tube having a diameter of 25 mm and a length of 125 mm. The plants were then cultured for 5 months under continuous lighting conditions (2500 lux) at 25°C. At the end of that period the plants obtained from each tube were aseptically separated into 10 groups, and each group transplanted into fresh culture medium for continuation of the culture, the whole subculture cycle being repeated 5 times. At the end of the subculture the contents of 20 test tubes were transplanted to a 8l-jar fermenter containing 6l of a liquid culture medium of the same composition as before, but without the agar, and with the sucrose concentration increased to 9% (w/v). The depth of the culture liquid at this stage was 23 cms. Cultivation was performed at 25°C at an aeration rate of 0.2 vvm for 60 days under conditions of continuous lighting (2500 lux) until the plants achieved an average length of 18 cms.

At that stage the aeration rate was increased to 1.5 vvm and the culture continued for one further month during which the liquid medium gradually evaporated so that the propagated plants were progressively exposed above the liquid level and into the aerial phase. At the end of one month, the volume of the culture medium remaining was 1.7l to a depth of 6 cms. At this stage approximately 60% of the plant material was in the aerial phase, but not blighted. From this material approximately 1800 konjak corms were recovered from each fermenter, total weight 1.2 kg with an average corm weight of 0.7 g. Of this number of corms the number of corms weighing 1 g or more was 270.

In contrast, repetition of the final stages of the cultivation process without changing the rate of aeration over a two month period produced only 47 konjak corms per fermenter.

### Example 5

Growth points from buds of jack-in-the-pulpit corms were aseptically dissected under a stereoscopic microscope and transplanted into 10 ml of a Murashige-Skoog medium having a composition of Table 1, contained in a 125 mm x 25 mm diameter test tube and cultivated at 25°C for 3 months under conditions of continuous lighting (2500 lux). The plants were serially subcultured in the manner described in Example 3. At the end of the subculture stage the propagated plant material from 3 flasks were transplanted to a 8l-jar fermenter containing 6l of a liquid culture medium having the compositions shown in Table 1 but without the agar and with a sucrose concentration increased to 9% (w/v) and to a depth of 23 cms. Cultivation was performed at 25°C at an aeration rate of 0.1 vvm for 40 days under conditions of continuous lighting (2500 lux) until the plants achieved an average length of 20 cms. Cultivation was then continued for a further month at an increased aeration rate of 1.5 vvm. Over this one month period the liquid medium gradually evaporated so that the propagated plant material was progressively exposed above the liquid medium and into the aerial phase. At the end of one month 2.0l of culture medium remained to a depth of 8 cms with about 80% of the plant material exposed in the aerial phase, and not blighted. 3600 jack-in-the-pulpit corms were obtained per jar fermenter, total weight 1.8 kg, and with an average corm weight at 0.5 g. Of this number, the number of corms weighing 1 g or more was 280.

Repetition of the final stages of the cultivation process, hut without increasing the aeration rate over a two month period produced only 430 jack-in-the-pulpit corms per fermenter, total weight, per fermenter 130 g, average weight 0.3 g. Hardly any corms were obtained weighing 1 g. or more.

### Example 6

The growth points from the buds of Chinese yam stalks were aseptically dissected under a stereoscopic microscope and transplanted into a 125 mm x 25 mm diameter test tube containing 10 ml of Murashige-Skoog medium having a composition of Table 1 but supplemented with 0.1 mg/l of naphthalene acetic acid and 0.1 mg/l of BA. Cultivation was carried out at 25°C under conditions of continuous lighting (2500 lux) for 5 months. The plants were then transplanted to a 300 ml-flask containing 100 ml of fresh liquid medium having the same composition as before but without the agar. Rotary spinner culture (180 r.p.m.) was then performed at 25°C under conditions of continuous lighting (2500 lux) for two months. The 120 plants thus obtained were separated into 10 groups, each of 12 plants, and each of which groups was then transplanted into separate 300 ml-flasks each containing 100 ml of fresh liquid medium having the same composition as before. Rotary spinner culture (180 r.p.m.) was performed on each flask at 25°C under conditions of continuous lighting (2500 lux) for two months to propagate the plants, that separation and subculture procedure being repeated a total of 3 times. At the end of the serial subculture the propagated plants from 3 flasks were transplanted into a 8l-jar fermenter containing 6l of fresh liquid medium to a depth of 23 cms. The composition of the medium was as shown in Table 1 but without the agar, and with the sucrose concentration increased to 9% (w/v). Cultivation was performed at 25°C at an aeration rate of 0.1 vvm for 2 months under conditions of continuous lighting (2500 lux) until the plants achieved an average length of about 24 cms. Cultivation was continued for further two months but with the aeration rate increased to 2 vvm. During this time the liquid medium gradually evaporated so that the propagated plants were progressively exposed above the liquid medium into the aerial phase. At the end of two months the amount of liquid medium reduced to 0.7 l at a depth of 3 cms. with about 85% of the whole plants exposed in the aerial phase, and not blighted. 1900 Chinese yam tuberous roots were obtained per jar fermenter, total weight per fermenter 0.4 kg, average tuber weight 0.2 g.

The final stages of the cultivation process were repeated over four months using a different batch of plants from the subculture but without increasing the aeration rate. In this case the amount of liquid medium remaining was 4.7l to a depth of 18 cms. The yield was reduced to 800 tuberous roots per fermenter, total weight 140 g and an average weight of 0.18 g.

### Example 7

The growth points of buds of Scopolia japonica rhizomes were aseptically dissected under a stereoscopic microscope to a size of about 3mm and transplanted into a 125 mm x 25 mm diameter test tube containing 10 ml of a Murashige-Skoog medium having the composition set out in Table 1. Cultivation was carried out at 25°C for 2 months under conditions of continuous lighting (2500 lux). The plants were then transplanted to a second test tube of the same size containing 10 ml of the same Murasbige-Skoog medium but supplemented by BA to a concentration of 1 mg/l.

Cultivation was continued at 25°C for 40 days under conditions of continuous lighting (2500 lux). At that stage the plants were aseptically separated into 10 groups, which groups were then each transplanted into fresh medium having the same composition as described above, and subcultured in a similar manner to further propagate the plants. 20 of the thus propagated plants were then transplanted into a 8l-jar fermenter containing 6l of fresh liquid medium to a depth of 23 cms. The liquid culture medium has the composition shown in Table 1, without the agar, but supplemented by 1 mg/l BA and with the concentration of sucrose increased to 9% (w/v).

Cultivation was performed at 25°C at an aeration rate 0.1 vvm for 2 months under conditions of continuous lighting (2500 lux) until the plants achieved an average length of 20 cms. At that stage the aeration rate was increased to 1 vvm and cultivation continued for a further one and a half months without changing the other conditions. During this time the liquid medium gradually evaporated so that at the end of the cultivation process the amount of liquid medium remaining was 2l to a depth of 8 cms, with about 50% of the whole plants exposed in the aerial phase, and not blighted. At this stage the plants were removed and the rhizomes formed on the basal parts of the plants recovered at a yield of 670 rhizomes per fermenter.

By contrast, if the final stages of the cultivation process are carried out over 3.5 months without changing the aeration rate, the yield of rhizomes reduces to 420 per fermenter.

## Claims

1. A method for the mass propagation of tuber-forming plants selected from the group consisting of potato, taro, konjak, jack-in-the pulpit, Scopolia japonica and Chinese yam by tissue culture, which comprises:
A) aseptically culturing in a culture medium and to an initial plant size not exceeding 10 cms plant tissue from the selected species, such tissue containing at least one growth point;
B) aseptically subdividing the cultured plant material obtained in step A) and transferring the subdivided material to a fresh liquid culture medium containing a suitable carbon source, at a concentration of 60 g/L or less;
C) continuing the culture of the subdivided plant material under an illuminance of 200 to 10,000 lux in the fresh culture medium to obtain a cultured plant material having an average plant size in the range 15 to 30 cms;
D) in the event that 90% of the cultured plant material obtained in step C) is not submerged in the culture medium, adding fresh culture liquid until 90% of the cultured plant material is so submerged;
E) increasing the concentration of the carbon source in the liquid culture medium to 60 to 150 g/L and continuing the culture, but under an illuminance of 200 lux or less;
F) progressively reducing the volume of the culture liquid throughout step E) until from 50 to 98% of the cultured plant material is above the level of the culture medium, thereby to establish a propagated plant material comprising the tubers, corms, rhizomes or other tuberous root tissue of the plant in question and located substantially in the air phase above the culture liquid;
G) recovering from the plant culture at the end of step F) the said tubers, corms, rhizomes or other tuberous root tissue

2. A method according to claim 1, characterised in that in step C) the subdivided plant material is cultured at a concentration of from 5 to 50 plant pieces per litre of culture medium.

3. A method according to claim 1 or 2, wherein the carbon source is glucose or sucrose.

4. A method according to claim 3, characterised in that in step C) the continued culture of the plant pieces is carried out at a glucose or sucrose concentration in the range 25 to 35 g/L, at a temperature in the range 10 to 35°C, an illuminance in the range 200 to 10000 lux, a pH in the range 4 to 8 and an aeration rate of from 0.01 to 0.5 vvm.

5. A method according to claim 3 or 4 characterised in that in step E), the glucose or sucrose concentration is increased to 80 to 100 g/L, and the continued culture carried out at a temperature in the range 10 to 35°C, an illuminance of 200 lux or less, and a pH in the range 4 to 8.

6. A method according to claim 5, characterised in that the illuminance in step E) is zero.

7. A method according to claim 3, 4, 5 or 6, characterised in that, in step F) the volume of the culture liquid is reduced by increasing the aeration rate to 0.8 to 2.2 vvm thereby to increase the rate of evaporation of the culture liquid.

8. A method according to claim 3, 4, 5 or 6, characterised in that in step F) the volume of the culture liquid is reduced by periodic decantation of the culture liquid.

9. A method according to claim 8, characterised in that the volume of the culture liquid is reduced by decantation of the culture liquid, in from 3 to 10 steps, with from 10 to 20% of the remaining volume of culture liquid being decanted in each step.

10. A method according to any one of claims 1 to 9, wherein the plant tissue used is from the potato plant, and the product recovered in step G comprises potato tubers.

## Patentansprüche

1. Verfahren zur Vermehrung in großem Maßstab von Knollen bildenden Pflanzen ausgewählt aus der Gruppe Kartoffeln, Colocasia esculenta ("taro", Amdophophallus konjak "konjak", geflecktem Aronstab, Scopolia japonica und Diosconea ("Chinese yam") durch Gewebekultur umfassend:
A) keimfreies Züchten des Pflanzengewebes der ausgewählten Species in einem Kulturmedium bis zu einer anfänglichen Pflanzengröße, die 10 cm nicht überschreitet, wobei das Gewebe zumindest einen Vegetationspunkt enthält,
B) keimfreies Aufteilen des gezüchteten, nach Schritt A) erhaltenen Pflanzenmaterials und Überführen des aufgeteilten Materials in ein neues flüssiges Kulturmedium, das eine geeignete Kohlenstoffquelle mit einer Konzentration von 60 g/L oder weniger enthält,
C) Weiterzüchten des aufgeteilten Pflanzenmaterials in dem neuen Kulturmedium bei einer Beleuchtungsstärke von 200 bis 10 000 Lux zum Erhalt eines vermehrten Pflanzenmaterials mit einer durchschnittlichen Pflanzengröße im Bereich von 15 bis 30 cm,
D) zugeben von neuer Kulturflüssigkeit im Falle, daß 90 % des gezüchteten, nach Schritt C) erhaltenen Pflanzenmaterials in das Kulturmedium nicht eintauchen, bis 90 % des gezüchteten Pflanzenmaterials eingetaucht sind,
E) steigern der Konzentration der Kohlenstoffquelle in dem flüssigen Kulturmedium auf 60 bis 150 g/L und Weiterzüchten, allerdings bei einer Beleuchtung von 200 Lux oder weniger,
F) fortschreitendes Reduzieren des Volumens der Kulturflüssigkeit während Schritt E) bis sich 50 bis 98 % des vermehrten Pflanzenmaterials über dem Spiegel des Kulturmediums und sich im wesentlichen in der Luftphase über der Kulturflüssigkeit befinden, wobei ein vermehrtes Pflanzenmaterial hergestellt wird, umfassend die Knollen, Corme, Rhizome oder ein anderes knollenförmiges Wurzelgewebe der fraglichen Pflanze,
G) Gewinnen der besagten Knollen, Corme, Rhizome oder des anderen knollenförmigen Wurzelgewebes aus der Pflanzenkultur am Ende von Schritt F).

2. Verfahren nach Anspruch 1, dadurch gekennzeichnet, daß bei Schritt C) das aufgeteilte Pflanzenmaterial bei einer Konzentration von 5 bis 50 Pflanzenstücken pro Liter Kulturmedium gezüchtet wird.

3. Verfahren nach Anspruch 1 oder 2, wobei die Kohlenstoffquelle Glucose oder Saccharose ist.

4. Verfahren nach Anspruch 3, dadurch gekennzeichnet, daß bei Schritt C) die fortgesetzte Vermehrung der Pflanzenstücke bei einer Glucose- oder Saccharosekonzentration im Bereich von 25 bis 35 g/L, einer Temperatur im Bereich von 10 bis 35°C, einer Beleuchtungsstärke im Bereich von 200 bis 10 000 Lux, einem pH-Wert im Bereich von 4 bis 8 und einer Belüftungsrate von 0,01 bis 0,5 vvm durchgeführt wird.

5. Verfahren nach Anspruch 3 oder 4, dadurch gekennzeichnet, daß bei Schritt E) die Glucose- oder Saccharosekonzentration auf 80 bis 100 g/L erhöht wird und die fortgesetzte Vermehrung bei einer Temperatur im Bereich von 10 bis 35°C, einer Beleuchtungsstärke von 200 Lux oder weniger und einem pH-Wert im Bereich von 4 bis 8 durchgeführt wird.

6. Verfahren nach Anspruch 5, dadurch gekennzeichnet, daß die Beleuchtung bei Schritt E) null ist.

7. Verfahren nach Anspruch 3, 4, 5 oder 6, dadurch gekennzeichnet, daß bei Schritt F) das Volumen der Kulturflüssigkeit durch Steigerung der Belüftungsrate auf 0,8 bis 2,2 vvm erniedrigt wird, um dadurch die Verdunstungsrate der Kulturflüssigkeit zu erhöhen.

8. Verfahren nach einem der Ansprüche 3, 4, 5 oder 6, dadurch gekennzeichnet, daß bei Schritt F) das Volumen der Kulturflüssigkeit durch regelmäßiges Dekantieren der Kulturflüssigkeit erniedrigt wird.

9. Verfahren nach Anspruch 8, dadurch gekennzeichnet, daß das Volumen der Kulturflüssigkeit in 3 bis 10 Schritten erniedrigt wird, wobei bei jedem Schritt 10 bis 20 % des verbliebenen Volumens der Kulturflüssigkeit dekantiert werden.

10. Verfahren nach einem der Ansprüche 1 bis 9, wobei das verwendete Pflanzengewebe von einer Kartoffelpflanze stammt und das gewonnene Produkt aus Schritt G) Kartoffelknollen umfaßt.

## Revendications

1. Procédé de multiplication de plantes formant des tubercules, choisies dans le groupe constitué par les pomme de terre, taro, konjak, arum, scopolie du Japon et igname de Chine, par culture de tissus, lequel procédé comporte les étapes suivantes :
A) culture aseptique, dans un milieu de culture et jusqu'à une taille initiale de plant ne dépassant pas 10 cm, d'un tissu de plante de l'espèce choisie, ce tissu contenant au moins un point de croissance ;
B) subdivision aseptique du matériau végétal cultivé, obtenu dans l'étape A), et transfert du matériau subdivisé dans un milieu de culture frais liquide, contenant une source appropriée de carbone en une concentration inférieure ou égale à 60 g/l ;
C) poursuite de la culture du matériau végétal subdivisé, sous un éclairement lumineux de 200 à 10 000 lux, dans le milieu de culture frais, pour obtenir un matériau végétal cultivé présentant une taille moyenne de plant située dans l'intervalle allant de 15 à 30 cm ;
D) dans le cas où 90 % du matériau végétal cultivé obtenu dans l'étape C) ne sont pas immergés dans le milieu de culture, addition de milieu de culture liquide frais jusqu'à ce que 90 % du matériau végétal cultivé soient immergés dedans ;
E) augmentation de la concentration de la source de carbone dans le milieu de culture liquide jusqu'à une valeur de 60 à 150 g/l, et poursuite de la culture, mais sous un éclairement lumineux inférieur ou égal à 200 lux ;
F) réduction progressive du volume de liquide de culture, tout au long de l'étape E), jusqu'à ce que 50 à 98 % du matériau végétal cultivé se trouvent au-dessus du niveau du milieu de culture, de façon à obtenir un matériau végétal qui s'est multiplié, comprenant des tubercules, cormus, rhizomes ou autres tissus de racines tubérisées de plante de l'espèce concernée, situés essentiellement dans la phase aérienne au-dessus du liquide de culture ;
G) récupération dans la culture de plants, à la fin de l'étape F), desdits tubercules, cormus, rhizomes ou autre tissus de racines tubérisées.

2. Procédé conforme à la revendication 1, caractérisé en ce que, dans l'étape C), le matériau végétal subdivisé est cultivé à une concentration de 5 à 50 morceaux de plante par litre de milieu de culture.

3. Procédé conforme à la revendication 1 ou 2, dans lequel la source de carbone est du glucose ou du saccharose.

4. Procédé conforme à la revendication 3, caractérisé en ce que, dans l'étape C), la culture des morceaux de plante est poursuivie avec une concentration de glucose ou de saccharose de 25 à 35 g/l, à une température de 10 à 35°C, sous un éclairement de 200 à 10 000 lux, à un pH de 4 à 8 et avec une vitesse d'aération de 0,01 à 0,5 vvm.

5. Procédé conforme à la revendication 3 ou 4, caractérisé en ce que, dans l'étape E), on augmente la concentration de glucose ou de saccharose à une valeur de 80 à 100 g/l, et l'on poursuit la culture à une température de 10 à 35°C, sous un éclairement inférieur ou égal à 200 lux et à un pH de 4 à 8.

6. Procédé conforme à la revendication 5, caractérisé en ce que l'éclairement lumineux est nul dans l'étape E).

7. Procédé conforme à la revendication 3, 4, 5 ou 6, caractérisé en ce que, dans l'étape F), on fait diminuer le volume de liquide de culture en augmentant la vitesse d'aération à une valeur de 0,8 à 2,2 vvm, pour augmenter ainsi la vitesse d'évaporation du liquide de culture.

8. Procédé conforme à la revendication 3, 4, 5 ou 6, caractérisé en ce que, dans l'étape F), on fait diminuer le volume de liquide de culture par soutirage périodique du liquide de culture.

9. Procédé conforme à la revendication 8, caractérisé en ce que l'on fait diminuer le volume de liquide de culture par soutirage du liquide de culture, en 3 à 10 étapes, en soutirant dans chaque étape de 10 à 20 % du volume restant de liquide de culture.

10. Procédé conforme à l'une quelconque des revendications 1 à 9, dans lequel le tissu végétal utilisé provient de plants de pomme de terre, et le produit récupéré dans l'étape G) comprend des tubercules de pomme de terre.
